# EUROPEAN PATENT APPLICATION

(11) **EP 4 583 638 A1**
(43) Date of publication of application: **09.07.2025**
(21) Application number: 24187153.2
(22) Date of filing: 08.07.2024
(51) Int. Cl.: H05H 3/06

(54) **BEAM SHAPING EQUIPMENT AND SUPPORTING STRUCTURE**

(30) Priority: 08.01.2024 TW 113100808
(71) Applicant: Heron Neutron Medical Corp., Zhubei City, Hsinchu County 302 (TW)
(72) Inventor: LU, Cheng-Ji, 302 Zhubei City (TW)
(74) Representative: Berggren Oy

(57) **Abstract**

A beam shaping equipment includes a beam shaping body and a main supporting frame. The beam shaping body includes a front module and a rear module. The rear module defines a working space with the front module therebetween. The working space is configured to accommodate a neutron source. The main supporting frame is configured to support the front module and the rear module. The neutron source is configured to generate a plurality of neutrons. The beam shaping body is configured to adjust an energy spectrum of the neutrons to generate a neutron beam.

## Description

### BACKGROUND

### Technical Field

The present disclosure relates to beam shaping equipment and supporting structures of these beam shaping equipment.

### Description of Related Art

Boron Neutron Capture Therapy (BNCT) is a targeted form of heavy-particle radiotherapy. The treatment device utilizing this technology generates ion beams through an accelerator to bombard a target material to produce a neutron source. A beam shaping assembly (BSA) is employed to collect, decelerate and converge to produce a highly efficient ion beam, so as to enhance the quality of treatment.

However, the accelerator and the beamline tube connected have a requirement of operation, maintenance and repair, and they are necessary to be separated from the beam shaping assembly, such that sufficient working space is provided between the beam shaping assembly and the combination of the accelerator and the beamline tube. After the completion of the operation, maintenance and repair, both of the beam shaping assembly and the combination of the accelerator and the beamline tube need to be restored to their original positions. Since both of the accelerator and the beam shaping assembly are heavy devices, alignment is uneasy. Therefore, the way to allow a user to carry out an efficient and accurate adjustment of position of the accelerator and the beam shaping assembly in a limited space in a simple and easy manner is undoubtedly an issue which the industry highly concerns.

### SUMMARY

A technical aspect of the present disclosure is to provide a beam shaping equipment, which can achieve a better space utilization and can accurately adjust the position and level of the beam shaping body, so that it can accurately align with the accelerator. Furthermore, during the installation or subsequent maintenance of the accelerator and the beamline tube connected, the front and rear modules of the beam shaping body can be easily and quickly separated to provide the required working space. After the completion of the operation, the front and rear modules of the beam shaping body can also be easily and quickly reset and assembled.

According to an embodiment of the present disclosure, a beam shaping equipment includes a beam shaping body and a main supporting frame. The beam shaping body includes a front module and a rear module. The rear module defines a working space with the front module therebetween. The working space is configured to accommodate a neutron source. The main supporting frame is configured to support the front module and the rear module. The neutron source is configured to generate a plurality of neutrons. The beam shaping body is configured to adjust an energy spectrum of the neutrons to generate a neutron beam.

In one or more embodiments of the present disclosure, the main supporting frame includes a first supporting bracket, a plurality of first tracks, a first limiting bracket, a plurality of anchor seats and a plurality of first level adjusting devices. The first tracks are disposed on the first supporting bracket. The front module and the rear module are respectively configured to be movably disposed on the first tracks. The first supporting bracket is movably connected with the first limiting bracket. The anchor seats are separated from each other and disposed on a stage. The first level adjusting devices are respectively connected between a corresponding one of the anchor seats and the first limiting bracket.

In one or more embodiments of the present disclosure, the first tracks respectively extend along a first direction. The first supporting bracket is configured to move along a first direction and a second direction relative to the first limiting bracket. The second direction is perpendicular to the first direction.

In one or more embodiments of the present disclosure, the first level adjusting devices are respectively configured to adjust a position of the first limiting bracket relative to a corresponding one of the anchor seats along a third direction. The third direction is perpendicular to the first direction and the second direction.

In one or more embodiments of the present disclosure, the beam shaping equipment further includes a first auxiliary supporting frame and a second auxiliary supporting frame. The first auxiliary supporting frame is detachably connected with the main supporting frame and the front module. The first auxiliary supporting frame is configured to move the front module relative to the main supporting frame. The second auxiliary supporting frame is detachably connected with the main supporting frame and the rear module. The second auxiliary supporting frame is configured to move the rear module relative to the main supporting frame.

In one or more embodiments of the present disclosure, the first tracks respectively extend along a first direction. The front module and the rear module are arranged along the first direction. The first auxiliary supporting frame and the second auxiliary supporting frame are detachably connected to opposite sides of the main supporting frame along the first direction.

In one or more embodiments of the present disclosure, the first auxiliary supporting frame includes a second supporting bracket, a plurality of second tracks, a second limiting bracket, a first base frame, a plurality of second level adjusting devices, a first driving portion and a first moving portion. The second supporting bracket is detachably connected with the first supporting bracket. The second tracks is disposed on the second supporting bracket and aligned with the first tracks. The second limiting bracket supports and fixes the second supporting bracket. The first base frame is detachably connected with at least a corresponding one of the anchor seats. The second level adjusting devices are respectively connected between the first base frame and the second limiting bracket. The second level adjusting devices are configured to adjust a distance between the first base frame and the second limiting bracket. The first driving portion is connected with the second supporting bracket. The first moving portion is configured to be detachably connected with the front module. The first driving portion is connected with the first moving portion and configured to drive the first moving portion to move.

In one or more embodiments of the present disclosure, the first driving portion and the first moving portion are located between the second tracks.

In one or more embodiments of the present disclosure, the first driving portion includes a first connecting piece, a first threaded rod and at least one first guiding rod. The first threaded rod is connected between the second supporting bracket and the first connecting piece. The first threaded rod is configured to rotate relative to the second supporting bracket and the first connecting piece. The first moving portion couples with the first threaded rod. The first guiding rod penetrates through the first moving portion and is connected between the second supporting bracket and the first connecting piece. The first guiding rod, the first threaded rod and the second tracks are parallel with each other.

In one or more embodiments of the present disclosure, the second auxiliary supporting frame includes a third supporting bracket, a plurality of third tracks, a third limiting bracket, a second base frame, a plurality of third level adjusting devices, a second driving portion and a second moving portion. The third supporting bracket is detachably connected with the first supporting bracket. The third tracks are disposed on the third supporting bracket and aligned with the first tracks. The third limiting bracket supports and fixes the third supporting bracket. The second base frame is detachably connected with at least another corresponding one of the anchor seats. The third level adjusting devices are respectively connected between the second base frame and the third limiting bracket. The third level adjusting devices are configured to adjust a distance between the second base frame and the third limiting bracket. The second driving portion is connected with the third supporting bracket. The second moving portion is configured to be detachably connected with the rear module. The second driving portion is connected with the second moving portion and configured to drive the second moving portion to move.

In one or more embodiments of the present disclosure, the second driving portion and the second moving portion are located between the third tracks.

In one or more embodiments of the present disclosure, the second driving portion includes a second connecting piece, a second threaded rod and at least one second guiding rod. The second threaded rod is connected between the third supporting bracket and the second connecting piece. The second threaded rod is configured to rotate relative to the third supporting bracket and the second connecting piece. The second moving portion couples with the second threaded rod. The second guiding rod penetrates through the second moving portion and is connected between the third supporting bracket and the second connecting piece. The second guiding rod, the second threaded rod and the third tracks are parallel with each other.

In one or more embodiments of the present disclosure, the first threaded rod has a first length. The second threaded rod has a second length. The second length is different from the first length.

According to an embodiment of the present disclosure, a supporting frame includes a main supporting frame. The main supporting frame includes a first limiting bracket, a first supporting bracket, a plurality of first tracks, a plurality of anchor seats and a plurality of first level adjusting devices. The first supporting bracket is movably connected with the first limiting bracket. The first tracks are disposed on the first supporting bracket. The anchor seats are separated from each other and disposed on a stage. The first level adjusting devices are respectively connected between a corresponding one of the anchor seats and the first limiting bracket.

In one or more embodiments of the present disclosure, the first tracks respectively extend along a first direction. The first supporting bracket is configured to move along a first direction and a second direction relative to the first limiting bracket. The second direction is perpendicular to the first direction.

In one or more embodiments of the present disclosure, the first level adjusting devices are respectively configured to adjust a position of the first limiting bracket relative to a corresponding one of the anchor seats along a third direction. The third direction is perpendicular to the first direction and the second direction.

In one or more embodiments of the present disclosure, the first tracks respectively extend along a first direction. The supporting structure further includes a first auxiliary supporting frame and a second auxiliary supporting frame. The first auxiliary supporting frame is detachably connected to a side of the main supporting frame along the first direction. The second auxiliary supporting frame detachably connected to another side of the main supporting frame along the first direction.

In one or more embodiments of the present disclosure, the first auxiliary supporting frame includes a second supporting bracket, a plurality of second tracks, a second limiting bracket, a first base frame, a plurality of second level adjusting devices, a first moving portion and a first driving portion. The second supporting bracket is detachably connected with the first supporting bracket. The second tracks are disposed on the second supporting bracket and aligned with the first tracks. The second limiting bracket supports and fixes the second supporting bracket. The first base frame is detachably connected with at least a corresponding one of the anchor seats. The second level adjusting devices are respectively connected between the first base frame and the second limiting bracket. The second level adjusting devices are respectively configured to adjust a distance between the first base frame and the second limiting bracket. The first driving portion is connected with the second supporting bracket and the first moving portion. The first driving portion is configured to drive the first moving portion to move.

In one or more embodiments of the present disclosure, the first driving portion and the first moving portion are located between the second tracks.

In one or more embodiments of the present disclosure, the first driving portion includes a first connecting piece, a first threaded rod and at least one first guiding rod. The first threaded rod is connected between the second supporting bracket and the first connecting piece. The first threaded rod is configured to rotate relative to the second supporting bracket and the first connecting piece. The first moving portion couples with the first threaded rod. The first guiding rod penetrates through the first moving portion and is connected between the second supporting bracket and the first connecting piece. The first guiding rod, the first threaded rod and the second tracks are parallel with each other.

In one or more embodiments of the present disclosure, the second auxiliary supporting frame includes a third supporting bracket, a plurality of third tracks, a third limiting bracket, a second base frame, a plurality of third level adjusting devices, a second moving portion and a second driving portion. The third supporting bracket is detachably connected with the first supporting bracket. The third tracks are disposed on the third supporting bracket and aligned with the first tracks. The third limiting bracket supports and fixes the third supporting bracket. The second base frame is detachably connected with at least another corresponding one of the anchor seats. The third level adjusting devices are respectively connected between the second base frame and the third limiting bracket. The third level adjusting devices are respectively configured to adjust a distance between the second base frame and the third limiting bracket. The second driving portion is connected with the third supporting bracket and the second moving portion. The second driving portion is configured to drive the second moving portion to move.

In one or more embodiments of the present disclosure, the second driving portion and the second moving portion are located between the third tracks.

In one or more embodiments of the present disclosure, the second driving portion includes a second connecting piece, a second threaded rod and at least one second guiding rod. The second threaded rod is connected between the third supporting bracket and the second connecting piece. The second threaded rod is configured to rotate relative to the third supporting bracket and the second connecting piece. The second moving portion couples with the second threaded rod. The second guiding rod penetrates through the second moving portion and is connected between the third supporting bracket and the second connecting piece. The second guiding rod, the second threaded rod and the third tracks are parallel with each other.

In one or more embodiments of the present disclosure, the first threaded rod has a first length. The second threaded rod has a second length. The second length is different from the first length.

The above-mentioned embodiments of the present disclosure have at least the following advantages:
(1) By connecting the first auxiliary supporting frame and the second auxiliary supporting frame to opposite sides of the main supporting frame along the first direction, the front module and the rear module can move relative to the main supporting frame, such that the front module and the rear module can be moved away from or towards each other. This can achieve a better utilization of space in the environment where the beam shaping equipment is located, making it convenient for users to operate, repair, or maintain the accelerator and the beamline tube connected. Moreover, when the first auxiliary supporting frame and the second auxiliary supporting frame no longer support the front module and the rear module, the first auxiliary supporting frame and the second auxiliary supporting frame can be removed, facilitating a saving of space.
(2) Since the movements of the front module and the rear module disposed on the main supporting frame along the first direction, the second direction and the third direction are achieved through mechanisms independent of each other, i.e., the movements of the front module and rear module along the first direction, the second direction and the third direction do not interfere with each other, a user can precisely adjust the positions and horizontal heights of the front module and rear module, thereby improving the operational efficiency of the beam shaping equipment.
(3) Since the maximum stroke of the first moving portion is different from the maximum stroke of the second moving portion, the ranges of the movements of the front module and the rear module in the first direction can be different, thereby enhancing the flexibility of utilization of the beam shaping equipment.

### BRIEF DESCRIPTION OF THE DRAWINGS

The disclosure can be more fully understood by reading the following detailed description of the embodiments, with reference made to the accompanying drawings as follows:
Fig. 1 is a schematic view of a beam shaping equipment according to an embodiment of the present invention;
Fig. 2 is a side view of the beam shaping equipment of Fig 1;
Fig. 3 is a schematic view of the beam shaping equipment of Figs. 1-2, in which the front module and the rear module are placed on the main supporting frame, while the first auxiliary supporting frame and the second auxiliary supporting frame are removed;
Fig. 4 is an exploded view of the main supporting frame of Fig. 3;
Fig. 5 is a schematic view of the first auxiliary supporting frame of Figs. 1-2; and
Fig. 6 is a schematic view of the second auxiliary supporting frame of Figs. 1-2.

### DETAILED DESCRIPTION

Drawings will be used below to disclose embodiments of the present disclosure. For the sake of clear illustration, many practical details will be explained together in the description below. However, it is appreciated that the practical details should not be used to limit the claimed scope. In other words, in some embodiments of the present disclosure, the practical details are not essential. Moreover, for the sake of drawing simplification, some customary structures and elements in the drawings will be schematically shown in a simplified way. Wherever possible, the same reference numbers are used in the drawings and the description to refer to the same or like parts.

Unless otherwise defined, all terms (including technical and scientific terms) used herein have the same meanings as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

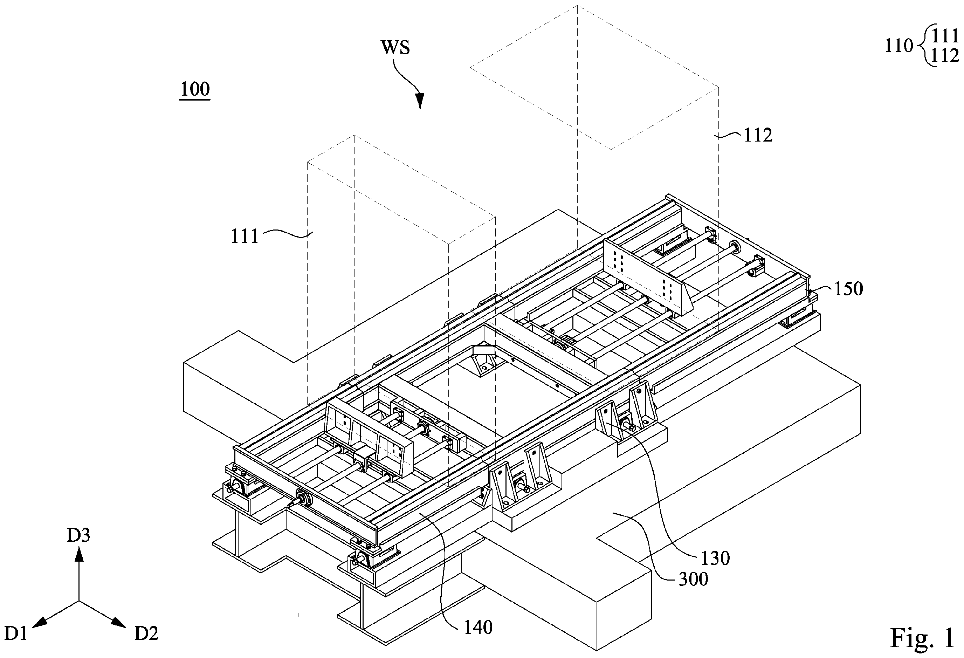

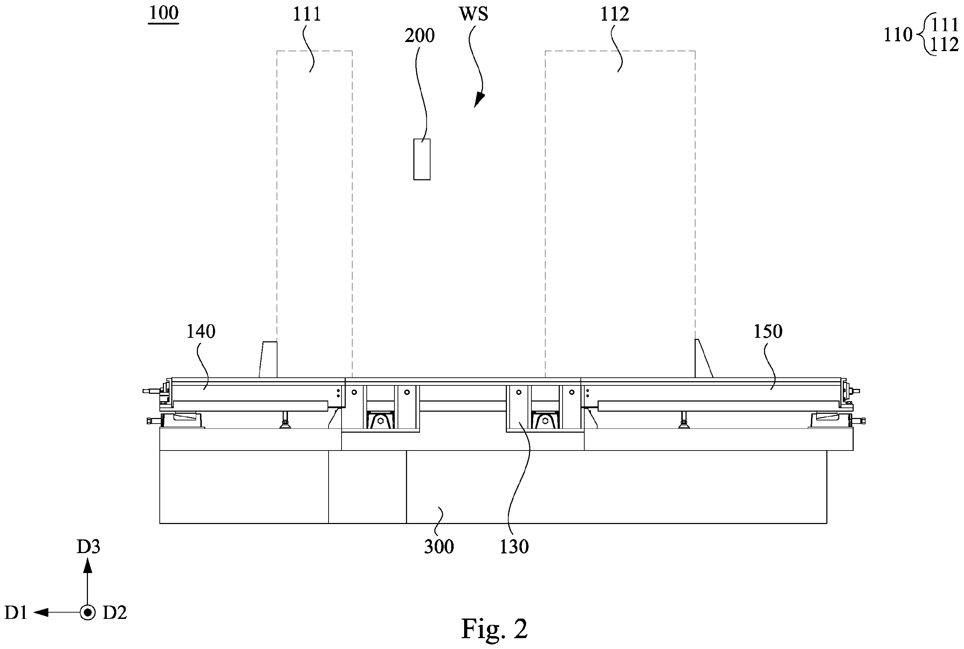

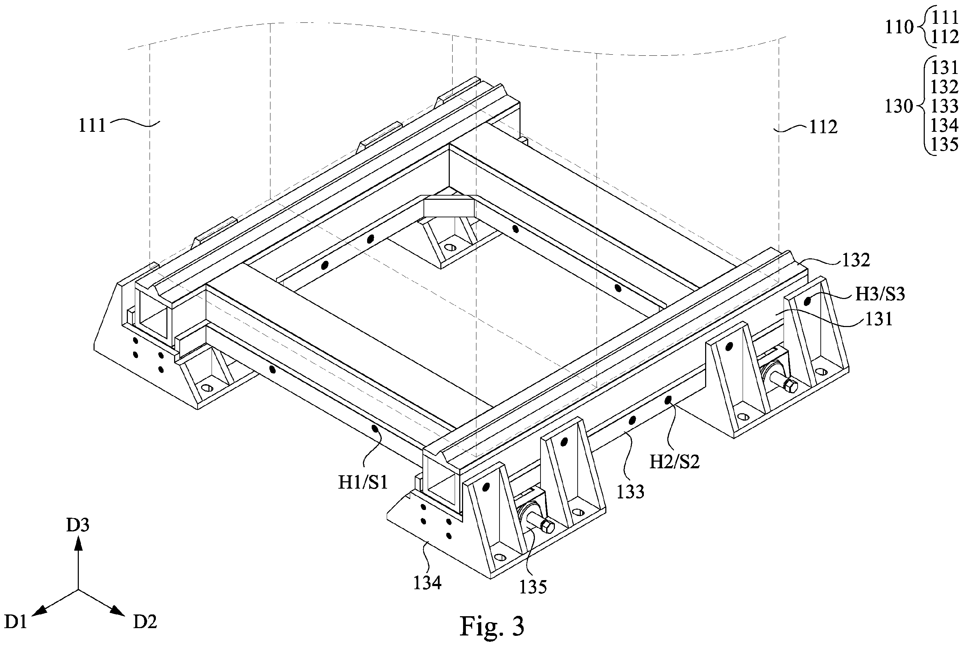

Reference is made to Figs. 1-2. Fig. 1 is a schematic view of a beam shaping equipment 100 according to an embodiment of the present invention. Fig. 2 is a side view of the beam shaping equipment 100 of Fig 1. In this embodiment, as shown in Figs. 1-2, a beam shaping equipment 100 includes a beam shaping body 110, a main supporting frame 130, a first auxiliary supporting frame 140, and a second auxiliary supporting frame 150. The beam shaping body 110 includes a front module 111 and a rear module 112. For the sake of drawing simplification, in Fig. 1, the front module 111 and the rear module 112 of the beam shaping body 110 are shown in dashed lines, and the rear module 112 and the front module 111 define a working space WS therebetween. The working space WS is configured to accommodate a neutron source 200 (please see Fig. 2 for the neutron source 200, the neutron source 200 is not shown in Fig. 1 for drawing simplification). The neutron source 200 is practically a target material. The neutron source 200 is connected with an accelerator through a beamline tube (the accelerator and the beamline tube are not shown). An ion beam is generated from the accelerator and impacts the neutron source 200 through the beamline tube to generate neutrons. During the operation of the beam shaping equipment 100, the front module 111 and the rear module 112 are connected and assembled (as shown in Fig. 3), and the beam shaping body 110 adjusts the energy spectrum of neutrons generated by the neutron source 200 by collecting, decelerating and converging, thereby producing a highly efficient neutron beam to enhance the therapeutic effectiveness of the beam shaping equipment 100. Furthermore, the main supporting frame 130 is configured to support the front module 111 and the rear module 112. The first auxiliary supporting frame 140 is detachably connected with the main supporting frame 130 and the front module 111, and is configured to move the front module 111 relative to the main supporting frame 130. The second auxiliary supporting frame 150 is detachably connected with the main supporting frame 130 and the rear module 112, and is configured to move the rear module 112 relative to the main supporting frame 130.

To be specific, as shown in Figs. 1-2, the front module 111 and the rear module 112 are arranged along a first direction D1, and the first auxiliary supporting frame 140 and the second auxiliary supporting frame 150 are detachably connected to opposite sides of the main supporting frame 130 along the first direction D1. Therefore, the front module 111 can move from the first auxiliary supporting frame 140 to the main supporting frame 130 or from the main supporting frame 130 to the first auxiliary supporting frame 140 along the first direction D1. Similarly, the rear module 112 can move from the second auxiliary supporting frame 150 to the main supporting frame 130 or from the main supporting frame 130 to the second auxiliary supporting frame 150 along the first direction D1. In this way, by connecting the first auxiliary supporting frame 140 and the second auxiliary supporting frame 150 to opposite sides of the main supporting frame 130 along the first direction D1, the front module 111 and the rear module 112 can move relative to the main supporting frame 130, such that the front module 111 and the rear module 112 can be moved away from or towards each other. This can achieve a better utilization of space in the environment where the beam shaping equipment 100 is located, making it convenient for users to operate, repair, or maintain the accelerator and the beamline tube connected. For example, the distance between the front module 111 and the rear module 112 can be up to 200 cm.

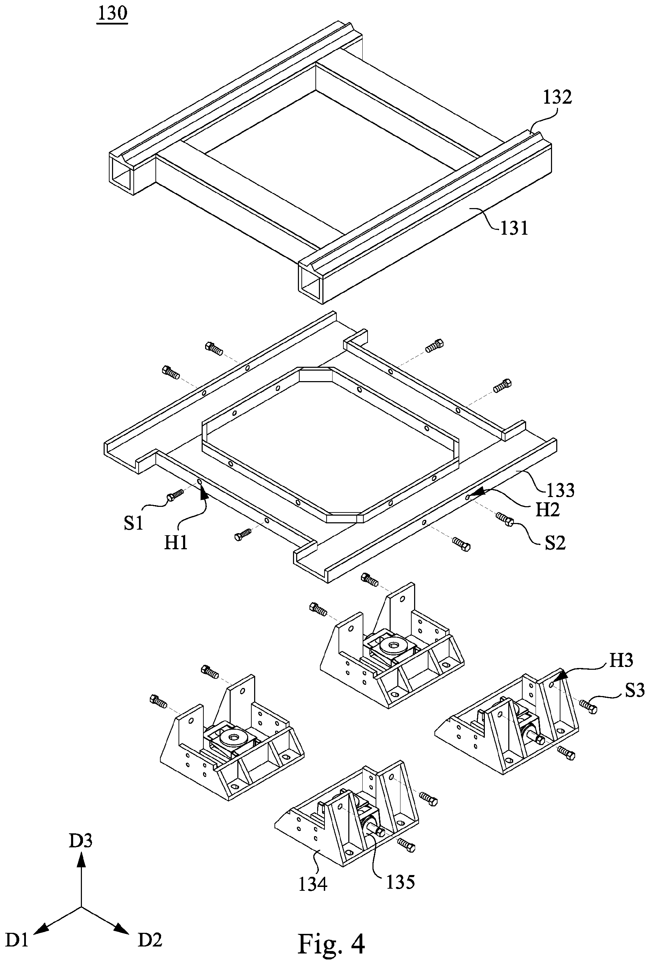

Reference is made to Figs. 3-4. Fig. 3 is a schematic view of the beam shaping equipment 100 of Figs. 1-2, in which the front module 111 and the rear module 112 are placed on the main supporting frame 130, while the first auxiliary supporting frame 140 and the second auxiliary supporting frame 150 are removed. Fig. 4 is an exploded view of the main supporting frame 130 of Fig. 3. For the sake of drawing simplification, in Fig. 3, the front module 111 and the rear module 112 of the beam shaping body 110 are shown in dashed lines, while the front module 111 and the rear module 112 are already moved towards each other and connected. The beam shaping body 110 already connected and assembled can adjust the energy spectrum of neutrons generated by the neutron source 200 to produce a highly efficient neutron beam. At this point, the first auxiliary supporting frame 140 and the second auxiliary supporting frame 150 (please see Figs. 1-2 for the first auxiliary supporting frame 140 and the second auxiliary supporting frame 150) no longer support the front module 111 and the rear module 112, such that the first auxiliary supporting frame 140 and the second auxiliary supporting frame 150 can be removed, facilitating a saving of space.

In this embodiment, as shown in Figs. 3-4, the main supporting frame 130 includes a first supporting bracket 131, a plurality of first tracks 132, a first limiting bracket 133, a plurality of anchor seats 134 and a plurality of first level adjusting devices 135. The first tracks 132 are disposed on the first supporting bracket 131. The front module 111 and rear module 112 are respectively configured to be movably disposed on the first tracks 132. The first supporting bracket 131 is movably connected with the first limiting bracket 133, and the first limiting bracket 133 is practically formed by channels welded together. The anchor seats 134 are separated from each other and disposed on a stage 300 (please see Figs. 1-2 for the stage 300). The first level adjusting devices 135 are respectively connected between a corresponding one of the anchor seats 134 and the first limiting bracket 133.

Furthermore, as shown in Figs. 3-4, the first tracks 132 respectively extend along the first direction D1, such that the front module 111 and rear module 112 can move on the first tracks 132 relative to the first supporting bracket 131 along the first direction D1. In addition, the first supporting bracket 131 is configured to move relative to the first limiting bracket 133 along the first direction D1 and a second direction D2, in which the second direction D2 is perpendicular to the first direction D1.

To be specific, as shown in Figs. 3-4, the first limiting bracket 133 has a plurality of first screw holes H1. The first screw holes H1 are distributed on both sides of the first limiting bracket 133 and respectively extend along the first direction D1. A user can use a plurality of first screws S1 to respectively couple with a corresponding one of the first screw holes H1 and press the first screws S1 against the first supporting bracket 131, thereby causing the first supporting bracket 131 to move relative to the first limiting bracket 133 along the first direction D1, which can achieve a precise movement of the front module 111 and rear module 112 disposed on the first supporting bracket 131 along the first direction D1, such that the front module 111 and rear module 112 can be accurately positioned relative to the neutron source 200. For example, a range of the movement of the first supporting bracket 131 relative to the first limiting bracket 133 along the first direction D1 is ±1 cm.

Similarly, as shown in Figs. 3-4, the first limiting bracket 133 also has a plurality of second screw holes H2. The second screw holes H2 are distributed on both sides of the first limiting bracket 133 and respectively extend along the second direction D2. A user can use a plurality of second screws S2 to respectively couple with corresponding one of the second screw holes H2 and press the second screws S2 against the first supporting bracket 131, thereby causing the first supporting bracket 131 to move relative to the first limiting bracket 133 along the second direction D2, which can achieve a precise movement of the front module 111 and rear module 112 disposed on the first supporting bracket 131 along the second direction D2, such that the front module 111 and rear module 112 can be accurately positioned relative to the neutron source 200.

Furthermore, as shown in Figs. 3-4, the anchor seats 134 respectively have a plurality of third screw holes H3. The third screw holes H3 respectively extend along the second direction D2. Under the condition that the first limiting bracket 133 is fixed relative to the anchor seats 134 along the second direction D2, a user can use a plurality of third screws S3 to respectively couple with a corresponding one of the third screw holes H3 and press the third screws S3 against the first supporting bracket 131, thereby causing the first supporting bracket 131 to move relative to the first limiting bracket 133 along the second direction D2, which can achieve a precise movement of the front module 111 and rear module 112 disposed on the first supporting bracket 131 along the second direction D2, such that the front module 111 and rear module 112 can be accurately positioned relative to the neutron source 200. For example, a range of the movement of the first supporting bracket 131 relative to the first limiting bracket 133 along the second direction D2 is ±1 cm.

However, it should be noted that, the method mentioned above of using screws to push the first supporting bracket 131 to move the first supporting bracket 131 relative to the first limiting bracket 133 is only illustrative and is not intended to limit the present disclosure. A person having ordinary skill in the art should appropriately choose the method of moving the first supporting bracket 131 relative to the first limiting bracket 133 according to the actual situation.

Furthermore, the first level adjusting devices 135 are respectively configured to adjust a position of the first limiting bracket 133 relative to a corresponding one of the anchor seats 134 along a third direction D3, and the third direction D3 is perpendicular to the first direction D1 and the second direction D2. By individually adjusting the position of the first limiting bracket 133 relative to the corresponding anchor seat 134 along the third direction D3 using the first level adjusting devices 135, a user can easily make the front module 111 and rear module 112 disposed on the main supporting frame 130 achieve accurate horizontal heights along the third direction D3 in a simple and easy manner, such that the front module 111 and rear module 112 can be accurately positioned relative to the neutron source 200. For example, a range of the movement of the first limiting bracket 133 relative to the corresponding anchor seat 134 along the third direction D3 is ±0.5 cm.

In this embodiment, the first tracks 132 on the first supporting bracket 131 respectively extend along the first direction D1, the coupling of the first screws S1 with the corresponding first screw holes H1 can also be finely adjusted along the first direction D1, the coupling of the second screws S2 with the corresponding second screw holes H2 and the coupling of the third screws S3 with the corresponding third screw holes H3 can be finely adjusted along the second direction D2, the first level adjusting devices 135 can finely adjust the position of the first limiting bracket 133 relative to a corresponding one of the anchor seats 134 along the third direction D3. Although the first screws S1 coupling with the first screw holes H1 can be finely adjusted along the first direction D1, in order to avoid interference between this adjustment and the coupling of the second screws S2 with the second screw holes H2 and/or the coupling of the third screws S3 with the third screw holes H3, the first tracks 132 must be moved along the first direction D1, such that the movements of different components of the main supporting frame 130 are independent of each other along the first direction D1, the second direction D2 and the third direction D3.

it is worth to note that, as mentioned above, since the movements of the front module 111 and the rear module 112 disposed on the main supporting frame 130 along the first direction D1, the second direction D2 and the third direction D3 are achieved through mechanisms independent of each other, i.e., the movements of the front module 111 and rear module 112 along the first direction D1, the second direction D2 and the third direction D3 do not interfere with each other, a user can precisely adjust the positions and horizontal heights of the front module 111 and rear module 112, thereby improving the operational efficiency of the beam shaping equipment 100.

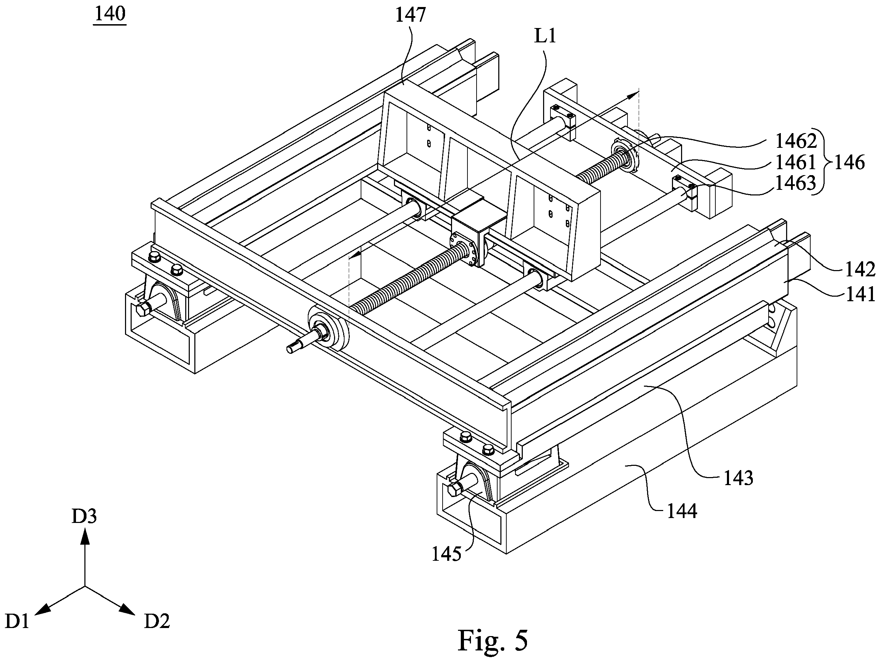

Reference is made to Fig. 5. Fig. 5 is a schematic view of the first auxiliary supporting frame 140 of Figs. 1-2. In this embodiment, as shown in Fig. 5, the first auxiliary supporting frame 140 includes a second supporting bracket 141, a plurality of second tracks 142, a second limiting bracket 143, a first base frame 144, a plurality of second level adjusting devices 145, a first driving portion 146 and a first moving portion 147. The second supporting bracket 141 is detachably connected with the first supporting bracket 131 (please see Figs. 1-2). The second tracks 142 are disposed on the second supporting bracket 141 and aligned with the first tracks 132 (please see Figs. 1-2). The second limiting bracket 143 supports and fixes the second supporting bracket 141. The first base frame 144 is detachably connected with at least a corresponding one of the anchor seats 134 (please see Figs 1-2). The second level adjusting devices 145 are respectively connected between the first base frame 144 and the second limiting bracket 143, and configured to move the second limiting bracket 143 relative to the first base frame 144 along the third direction D3, thereby adjusting the distance between the first base frame 144 and the second limiting bracket 143. The first driving portion 146 is connected with the second supporting bracket 141. For example, in this embodiment, the first driving portion 146 can further be detachably connected with the first supporting bracket 131. However, in other embodiments, according to the actual situation, the first driving portion 146 and the first supporting bracket 131 are separated from each other. This means the first driving portion 146 and the first supporting bracket 131 are not connected with each other. The first moving portion 147 is configured to be detachably connected with the front module 111 (please see Figs. 1-2). The first driving portion 146 is connected with the first moving portion 147 and is configured to drive the first moving portion 147 to move.

Furthermore, the first driving portion 146 and the first moving portion 147 are located between the second tracks 142. This means the second tracks 142 are separated from each other, thereby providing more stable support to the front module 111.

To be more specific, the first driving portion 146 includes a first connecting piece 1461, a first threaded rod 1462 and at least one first guiding rod 1463. As mentioned above, for example, in this embodiment, the first connecting piece 1461 is detachably connected with the first supporting bracket 131. However, in other embodiments, according to the actual situation, the first connecting piece 1461 and the first supporting bracket 131 are separated from each other. This means the first connecting piece 1461 and the first supporting bracket 131 are not connected with each other. The first threaded rod 1462 is connected between the second supporting bracket 141 and the first connecting piece 1461. The first threaded rod 1462 is configured to rotate relative to the second supporting bracket 141 and the first connecting piece 1461. The first moving portion 147 is coupled with the first threaded rod 1462. The first guiding rod 1463 penetrates through the first moving portion 147 and is connected between the second supporting bracket 141 and the first connecting piece 1461. The first guiding rod 1463, the first threaded rod 1462 and the second tracks 142 are parallel with each other and respectively extend along the first direction D1. By rotating the first threaded rod 1462 and simultaneously being restricted and guided by the first guiding rod 1463, the first moving portion 147 can move along the first guiding rod 1463 and the first threaded rod 1462, thereby moving the front module 111 connected with the first moving portion 147 along the first direction D1.

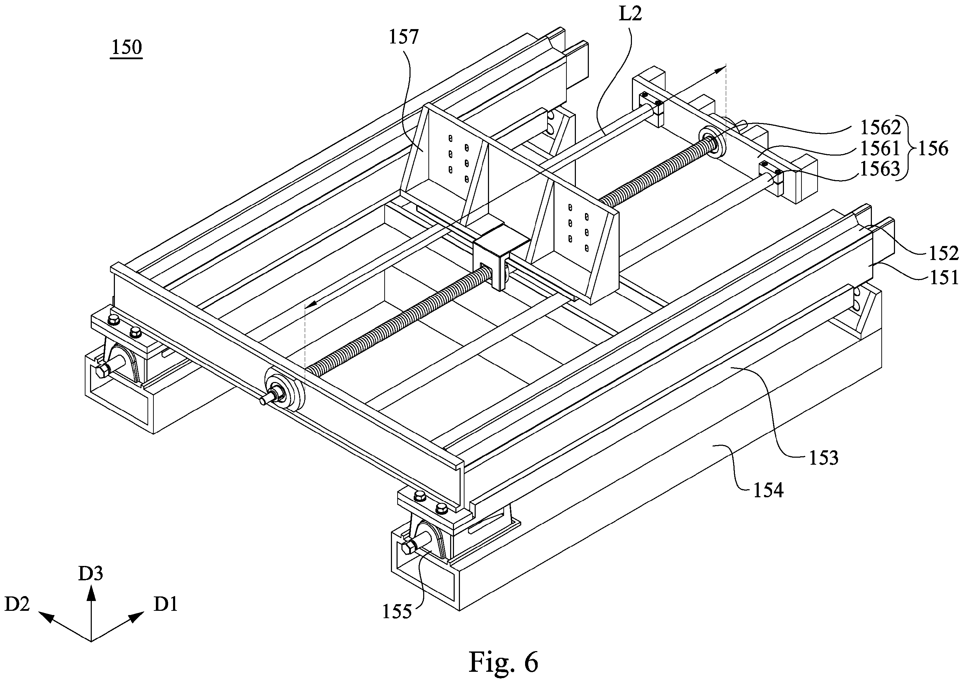

Reference is made to Fig. 6. Fig. 6 is a schematic view of the second auxiliary supporting frame 150 of Figs. 1-2. In this embodiment, as shown in Fig. 6, the second auxiliary supporting frame 150 includes a third supporting bracket 151, a plurality of third tracks 152, a third limiting bracket 153, a second base frame 154, a plurality of third level adjusting devices 155, a second driving portion 156 and a second moving portion 157. The third supporting bracket 151 is detachably connected with the first supporting bracket 131 (please see Figs. 1-2). The third tracks 152 are disposed on the third supporting bracket 151 and aligned with the first tracks 132 (please see Figs 1-2). The third limiting bracket 153 supports and fixes the third supporting bracket 151. The second base frame 154 is detachably connected with at least another corresponding one of the anchor seats 134 (please see Figs. 1-2). The third level adjusting devices 155 are respectively connected between the second base frame 154 and the third limiting bracket 153, and configured to move the third limiting bracket 153 relative to the second base frame 154 along the third direction D3, thereby adjusting the distance between the second base frame 154 and the third limiting bracket 153. The second driving portion 156 is connected with the third supporting bracket 151. For example, in this embodiment, the second driving portion 156 is detachably connected with the first supporting bracket 131. However, in other embodiments, according to the actual situation, the second driving portion 156 and the first supporting bracket 131 are separated from each other. This means the second driving portion 156 and the first supporting bracket 131 are not connected with each other. The second moving portion 157 is configured to be detachably connected with the rear module 112 (please see Figs. 1-2). The second driving portion 156 is connected with the second moving portion 157 and is configured to drive the second moving portion 157 to move.

Furthermore, the second driving portion 156 and the second moving portion 157 are located between the third tracks 152. This means the third tracks 152 are separated from each other, thereby providing more stable support to the rear module 112.

To be more specific, the second driving portion 156 includes a second connecting piece 1561, a second threaded rod 1562 and at least one second guiding rod 1563. As mentioned above, for example, in this embodiment, the second connecting piece 1561 is detachably connected with the first supporting bracket 131. However, in other embodiments, according to the actual situation, the second connecting piece 1561 and the first supporting bracket 131 are separated from each other. This means the second connecting piece 1561 and the first supporting bracket 131 are not connected with each other. The second threaded rod 1562 is connected between the third supporting bracket 151 and the second connecting piece 1561. The second threaded rod 1562 is configured to rotate relative to the third supporting bracket 151 and the second connecting piece 1561. The second moving portion 157 is coupled with the second threaded rod 1562. The second guiding rod 1563 penetrates through the second moving portion 157 and is connected between the third supporting bracket 151 and the second connecting piece 1561. The second guiding rod 1563, the second threaded rod 1562 and the third tracks 152 are parallel with each other and respectively extend along the first direction D1. By rotating the second threaded rod 1562 and simultaneously being restricted and guided by the second guiding rod 1563, the second moving portion 157 can move along the second guiding rod 1563 and the second threaded rod 1562, thereby moving the rear module 112 connected with the second moving portion 157 along the first direction D1.

Furthermore, as shown in Fig. 5, the first threaded rod 1462 has a first length L1, and as shown in Fig. 6, the second threaded rod 1562 has a second length L2. In practical applications, the first length L1 and the second length L2 are different. This means the maximum stroke of the first moving portion 147 is different from the maximum stroke of the second moving portion 157. In this way, the ranges of the movements of the front module 111 and the rear module 112 in the first direction D1 can be different, thereby enhancing the flexibility of utilization of the beam shaping equipment 100. For example, according to the actual situation, the second length L2 can be greater than the first length L1. This means the maximum stroke of the rear module 112 along the first direction D1 is greater than the maximum stroke of the front module 111 along the first direction D1.

In conclusion, the aforementioned embodiments of the present disclosure have at least the following advantages:
(1) By connecting the first auxiliary supporting frame and the second auxiliary supporting frame to opposite sides of the main supporting frame along the first direction, the front module and the rear module can move relative to the main supporting frame, such that the front module and the rear module can be moved away from or towards each other. This can achieve a better utilization of space in the environment where the beam shaping equipment is located, making it convenient for users to operate, repair, or maintain the accelerator and the beamline tube connected. Moreover, when the first auxiliary supporting frame and the second auxiliary supporting frame no longer support the front module and the rear module, the first auxiliary supporting frame and the second auxiliary supporting frame can be removed, facilitating a saving of space.
(2) Since the movements of the front module and the rear module disposed on the main supporting frame along the first direction, the second direction and the third direction are achieved through mechanisms independent of each other, i.e., the movements of the front module and rear module along the first direction, the second direction and the third direction do not interfere with each other, a user can precisely adjust the positions and horizontal heights of the front module and rear module, thereby improving the operational efficiency of the beam shaping equipment.
(3) Since the maximum stroke of the first moving portion is different from the maximum stroke of the second moving portion, the ranges of the movements of the front module and the rear module in the first direction can be different, thereby enhancing the flexibility of utilization of the beam shaping equipment.

## Claims

1. A beam shaping equipment (100), **characterized in that**, comprising:
a beam shaping body (110), comprising:
a front module (111); and
a rear module (112) defining a working space (WS) with the front module (111) therebetween, the working space (WS) being configured to accommodate a neutron source (200); and
a main supporting frame (130) configured to support the front module (111) and the rear module (112),
wherein the neutron source (200) is configured to generate a plurality of neutrons, and the beam shaping body (110) is configured to adjust an energy spectrum of the neutrons to generate a neutron beam.

2. The beam shaping equipment (100) of claim 1, **characterized in that**, the main supporting frame (130) comprises:
a first supporting bracket (131);
a plurality of first tracks (132) disposed on the first supporting bracket (131), the front module (111) and the rear module (112) are respectively configured to be movably disposed on the first tracks (132);
a first limiting bracket (133), the first supporting bracket (131) is movably connected with the first limiting bracket (133);
a plurality of anchor seats (134) separated from each other and disposed on a stage (300); and
a plurality of first level adjusting devices (135) respectively connected between a corresponding one of the anchor seats (134) and the first limiting bracket (133).

3. The beam shaping equipment (100) of claim 2, **characterized in that**, the first tracks (132) respectively extend along a first direction (D1), the first supporting bracket (131) is configured to move along a first direction (D1) and a second direction (D2) relative to the first limiting bracket (133), the second direction (D2) is perpendicular to the first direction (D1).

4. The beam shaping equipment (100) of claim 3, **characterized in that**, the first level adjusting devices (135) are respectively configured to adjust a position of the first limiting bracket (133) relative to a corresponding one of the anchor seats (134) along a third direction (D3), the third direction (D3) is perpendicular to the first direction (D1) and the second direction (D2).

5. The beam shaping equipment (100) of claim 2, **characterized in that**, further comprising:
a first auxiliary supporting frame (140) detachably connected with the main supporting frame (130) and the front module (111), and configured to move the front module (111) relative to the main supporting frame (130); and
a second auxiliary supporting frame (150) detachably connected with the main supporting frame (130) and the rear module (112), and configured to move the rear module (112) relative to the main supporting frame (130).

6. The beam shaping equipment (100) of claim 5, **characterized in that**, the first tracks (132) respectively extend along a first direction (D1), the front module (111) and the rear module (112) are arranged along the first direction (D1), the first auxiliary supporting frame (140) and the second auxiliary supporting frame (150) are detachably connected to opposite sides of the main supporting frame (130) along the first direction (D1).

7. The beam shaping equipment (100) of claim 5, **characterized in that**, the first auxiliary supporting frame (140) comprises:
a second supporting bracket (141) detachably connected with the first supporting bracket (131);
a plurality of second tracks (142) disposed on the second supporting bracket (141) and aligned with the first tracks (132);
a second limiting bracket (143) supporting and fixing the second supporting bracket (141);
a first base frame (144) detachably connected with at least a corresponding one of the anchor seats (134);
a plurality of second level adjusting devices (145) respectively connected between the first base frame (144) and the second limiting bracket (143), and configured to adjust a distance between the first base frame (144) and the second limiting bracket (143);
a first driving portion (146) connected with the second supporting bracket (141); and
a first moving portion (147) configured to be detachably connected with the front module (111), the first driving portion (146) is connected with the first moving portion (147) and configured to drive the first moving portion (147) to move.

8. The beam shaping equipment (100) of claim 7, **characterized in that**, the first driving portion (146) and the first moving portion (147) are located between the second tracks (142).

9. The beam shaping equipment (100) of claim 7, **characterized in that**, the first driving portion (146) comprises:
a first connecting piece (1461);
a first threaded rod (1462) connected between the second supporting bracket (141) and the first connecting piece (1461), and configured to rotate relative to the second supporting bracket (141) and the first connecting piece (1461), the first moving portion (147) couples with the first threaded rod (1462); and
at least one first guiding rod (1463) penetrating through the first moving portion (147) and connected between the second supporting bracket (141) and the first connecting piece (1461), the first guiding rod (1463), the first threaded rod (1462) and the second tracks (142) are parallel with each other.

10. The beam shaping equipment (100) of claim 9, **characterized in that**, the second auxiliary supporting frame (150) comprises:
a third supporting bracket (151) detachably connected with the first supporting bracket (131);
a plurality of third tracks (152) disposed on the third supporting bracket (151) and aligned with the first tracks (132);
a third limiting bracket (153) supporting and fixing the third supporting bracket (151);
a second base frame (154) detachably connected with at least another corresponding one of the anchor seats (134);
a plurality of third level adjusting devices (155) respectively connected between the second base frame (154) and the third limiting bracket (153), and configured to adjust a distance between the second base frame (154) and the third limiting bracket (153);
a second driving portion (156) connected with the third supporting bracket (151); and
a second moving portion (157) configured to be detachably connected with the rear module (112), the second driving portion (156) is connected with the second moving portion (157) and configured to drive the second moving portion (157) to move.

11. The beam shaping equipment (100) of claim 10, **characterized in that**, the second driving portion (156) and the second moving portion (157) are located between the third tracks (152).

12. The beam shaping equipment (100) of claim 10, **characterized in that**, the second driving portion (156) comprises:
a second connecting piece (1561);
a second threaded rod (1562) connected between the third supporting bracket (151) and the second connecting piece (1561), and configured to rotate relative to the third supporting bracket (151) and the second connecting piece (1561), the second moving portion (157) couples with the second threaded rod (1562); and
at least one second guiding rod (1563) penetrating through the second moving portion (157) and connected between the third supporting bracket (151) and the second connecting piece (1561), the second guiding rod (1563), the second threaded rod (1562) and the third tracks (152) are parallel with each other.

13. The beam shaping equipment (100) of claim 12, **characterized in that**, the first threaded rod (1462) has a first length (L1), the second threaded rod (1562) has a second length (L2), the second length (L2) is different from the first length (L1).

14. A supporting structure, **characterized in that**, comprising:
a main supporting frame (130), comprising:
a first limiting bracket (133);
a first supporting bracket (131) movably connected with the first limiting bracket (133);
a plurality of first tracks (132) disposed on the first supporting bracket (131);
a plurality of anchor seats (134) separated from each other and disposed on a stage (300); and
a plurality of first level adjusting devices (135) respectively connected between a corresponding one of the anchor seats (134) and the first limiting bracket (133).

15. The supporting structure of claim 14, wherein the first tracks (132) respectively extend along a first direction (D1), the supporting structure further comprises:
a first auxiliary supporting frame (140) detachably connected to a side of the main supporting frame (130) along the first direction (D1); and
a second auxiliary supporting frame (150) detachably connected to another side of the main supporting frame (130) along the first direction (D1).
